# EUROPÄISCHE PATENTSCHRIFT

(11) **EP 0 722 719 B1**
(45) Veröffentlichungstag und Bekanntmachung des Hinweises auf die Patenterteilung: **31.08.2005**
(21) Anmeldenummer: 96100528.7
(22) Anmeldetag: 16.01.1996
(51) Int. Cl.: A61K 9/18, A61K 35/78

(54) **Verfahren zur Herstellung von Flavano-Lignan-Zubereitungen**
Process for preparing flavano-lignan preparations
Procédé de fabrication de préparations à base de flavano-lignanes

(30) Priorität: 18.01.1995 DE 19501266
(43) Veröffentlichungstag der Anmeldung: 24.07.1996
(73) Patentinhaber: MADAUS AG, 51067 Köln (DE)
(72) Erfinder: Wächter, Wilfried, Dr. rer. nat., D-51469 Bergisch Gladbach (DE); Zaeske, Helga, D-51491 Overath (DE)
(74) Vertreter: Wolff, Felix

(56) Entgegenhaltungen:
- EP-A- 0 497 162
- EP-A- 0 577 143
- EP-A- 0 664 131
- FR-A- 2 350 098

## Beschreibung

Die Erfindung betrifft ein Verfahren zur Herstellung von Flavano-Lignan-Zubereitungen mit im Vergleich zu reinen Flavano-Lignanen verbesserter Freisetzung und Resorbierbarkeit, danach erhältliche Zubereitungen und diese enthaltende Arzneimittel zur Verwendung in der Therapie und Prophylaxe von Lebererkrankungen.

Aus der Mariendistel [Silybum marianum, Compositae] gewonnenes Silymarin ist seit langem bekannt. Verfahren zur Gewinnung von Polyhydroxyphenylchromanonen (Silymarin I-IV) und diese als Gemisch enthaltende Arzneimittel sind in der DE-C3-19 23 082 beschrieben.

Aus dieser Patenschrift ist auch bekannt, daß Silymarin verschiedene Inhaltsstoffe enthält. Den Hauptbestandteil in der Größenordnung von 80 bis 85 Mol-% bildet dabei ein Gemisch aus Silibinin und Isosilybin, wobei es sich um ein Gemisch aus Isomeren mit gleicher Summenformel [C₂₅H₂₂O₁₀] handelt. Weitere Inhaltsstoffe sind Silydianin [C₂₅H₂₂O₁₀] und Silychristin [C₂₅H₂₂O₁₀], bei denen es sich um Strukturisomere des Silybins handelt. Silibinin, Isosilybin, Silydianin und Silychristin zählen zur Gruppe der Flavano-Lignane, in denen Taxifolin mit Coniferyl-Alkohol verknüpft ist.

Aus der DE-C2-29 14 330 sind ein Verfahren zur Gewinnung von reinem Silymarin (Silymarin I-IV), nach diesem Verfahren gewonnenes Silymarin (I-IV) mit mindestens 90-%iger Reinheit und dessen Verwendung zur Behandlung von Lebererkrankungen bekannt.

Femer sind aus der DE-C2-35 37 656 ein Verfahren zur Gewinnung von isosylibinfreiem Silibinin sowie dieses enthaltende Arzneimittel bekannt.

Außerdem ist aus der FR-A-2 350 098 die Herstellung wasserlöslicher Polyhxdroxyphenylchromanon-Verbindungen aus Silybum marianum bekannt, die für Injektionszwecke bzw. für trinkbare Lösungen verwendet werden sollen. Dazu werden Lösungen von Polyhydroxyphenylchromanon in Dioxan hergestellt, mit wäßrigen Lösungen von Polyvinylpyrrolidonen versetzt und die so erhaltenen Lösungen gefriergetrocknet.

In der EP-A-0 577 143 wird beschrieben, wie Flavanoide aus-Gingko biloba-Extrakten, hier die Stoffgruppe der Flavonglukoside, mit Hilfe von Gelatine in molekular-disperser form in eine resorbierbare Zubereitung gebracht werden.

Es ist bekannt, daß Flavano-Lignane in der Regel in Wasser unlöslich oder zumindest schwer löslich sind. Aufgrund dieses Lösungsverhaltens ist die Freisetzungsrate dieser Verbindungen und damit auch ihre Bioverfügbarkeit und ihre Resorbierbarkeit im Körper des damit behandelten Menschen oder Säugetieres unbefriedigend. In der Vergangenheit wurde deshalb versucht, bei Flavano-Lignanen durch Behandlung bzw. Umsetzung mit geeigneten chemischen Agentien eine molekulare Veränderung herbeizuführen und sie so in Derivate mit verbesserter Wasserlöslichkeit und erhöhter Freisetzungsrate überzuführen. Zu den auf diese Weise gebildeten Derivaten zählen beispielsweise Addukte unter anderem mit Cyclodextrin, wie Silibinin-β-CD (CD = Cyclodextrin); Komplexverbindungen, z.B. mit Phosphatidylcholin oder mit bestimmten Aminozuckern; Ester, insbesondere mit Dicarbonsäuren, sowie Einschlußverbindungen. Nachteilig bei diesen Derivaten wirkt es sich aus, daß das im Einzelfall in Betracht kommende Flavano-Lignan an eine chemische Verbindung gebunden ist, die physiologisch als Fremdsubstanz wirken kann und möglicherweise unerwünschte Nebenreaktionen hervorruft oder die Wirksamkeit des Flavano-Lignans beeinträchtigt. Bei der Herstellung von Addukten, Komplexen oder Derivaten öffnet sich oft, z. B. im alkalischen Milieu, der γ-Pyronring des Flavanoanteils. Bei den genannten Derivaten besteht außerdem die Gefahr, daß bei der Verabreichung eines in der erwähnten Weise modifizierten bzw. derivatisierten Flavano-Lignans dieses zwar verbessert resorbiert wird, jedoch eine Wirkung verursacht, die von der für das Flavano-Lignan erwarteten und erwünschten wesentlich verschieden ist.

Aufgabe der Erfindung ist es, Flavano-Lignan-Zubereitungen zu schaffen, die keine Bindung der Flavano-Lignane an Fremdverbindungen aufweisen und eine gute hohe Freisetzungsrate aufweisen, durch die eine ausreichende Bioverfügbarkeit und eine gute Resorption durch den Körper des behandelten Individuums nach ihrer Verabreichung gewährleistet wird. Diese Flavano-Lignan-Zubereitungen sollen die erwünschten und erwarteten Wirkungen der betreffenden Flavano-Lignane aufweisen und keinerlei auf die Bindung an Fremdverbindungen, d. h. die bei der oben beschriebenen Derivatisierung verwendeten Agentien, zurückzuführende unerwünschte Nebenwirkungen oder Veränderung ihrer spezifischen Wirksamkeit oder ihres Wirkungsspektrums zeigen.

Die gestellte Aufgabe wird mittels eines Verfahrens gelöst, das dadurch gekennzeichnet ist, daß man a) eine wäßrig-alkoholische Lösung von pharmazeutisch annehmbaren Trägerstoffen und Netzmitteln herstellt, in dieser Lösung ein Flavano-Lignan suspendiert und das erhaltene Gemisch bis zur Siedetemperatur erwärmt, um eine klare Lösung zu bilden oder b) ein Flavano-Lignan und ein Netzmittel in Alkohol suspendiert, die erhaltene Suspension unter Rühren bis zur Bildung einer klaren Lösung erwärmt, diese mit einer wäßrigen Lösung von pharmazeutisch annehmbaren Trägerstoffen versetzt und die erhaltene Mischung solange unter Rühren erwärmt, bis eine klare Lösung vorliegt und daß man anschließend die nach a) oder b) erhaltene klare Lösung zur Bildung eines Copräzipitates konzentriert, filtriert und das entstandene Copräzipitat unter Vakuum trocknet.

Besonders bevorzugt ist die Herstellung von Flavano-Lignan-Zubereitungen, bei denen das Flavano-Lignan Silymarin oder Silibinin ist, insbesondere pulverförmiges Silymarin-Copräzipitat und/oder pulverförmiges Silibinin-Copräzipitat.

Bevorzugt verwendete pharmazeutisch annehmbare Trägerstoffe sind in Wasser lösliche Zuckerderivate wie Mono- und Disaccharide, Polyglykose, Polymaltose, Zuckeralkohole und/oder Cellulosen und Gemische hiervon. Als Mono- und Disaccharide sind besonders bevorzugt: Dextrose, Fructose, Galaktose, Glucose, Lactose, Mannose, Maltose, Saccharose, Xylose, Mannitol, Sorbitol. Von den Cellulosen sind bevorzugt: Hydroxyethylstärke, Carboxymethylcellulose, Natriumcarboxymethylstärke. Ausgewählte Anteile dieser Gruppe sind 2 - 80 %, bevorzugt 5 - 20 % (bezogen auf eingesetzte Feststoffe im Endprodukt einschließlich Wirkstoff). Die bevorzugten Trägerstoffe werden zusammen mit pharmazeutischen Netzmitteln und pharmazeutischen Trägerstoffen für disperse Systeme eingesetzt.

Bevorzugte pharmazeutische Netzmittel sind Polyoxyethylen-Derivate der Sorbitanester wie die Polysorbate 20, 40, 60 und 80, insbesondere Polysorbat 80 sowie auch Sorbitanester von Fettsäuren wie Sorbitanlaurat, -oleat, -palmitat und -sesquioleat. Ausgewählte Anteile davon sind 0,1 - 5 %, bevorzugt 0,5 - 2 %.

Bevorzugte pharmazeutische Trägerstoffe für disperse Systeme sind lineare Polymere des 1-Vinyl-2-pyrrolidon, insbesondere Poly[(2-oxo-1-pyrrolidinyl)ethylen], auch Polyvidon genannt. Ausgewählte Anteile sind 5 - 90 %, bevorzugt 20 - 50 %.

Nach der Herstellungsvariante a) werden die pharmazeutisch annehmbaren Trägerstoffe, die pharmazeutischen Netzmittel und die pharmazeutischen Trägerstoffe für disperse Synthese in dem angegebenen Verhältnis in Alkohol/Wasser gelöst und in dieser Lösung ein Flavano-Lignan suspendiert, wobei die Suspension bis zur Siedetemperatur erwärmt wird und eine klare Lösung bildet. Entsprechend - wie vorher beschrieben - wird die Herstellungsvariante b) durchgeführt. Anschließend wird ein Copräzipitat durch Konzentration gebildet, abfiltriert und im Vakuum getrocknet.

Bei der Herstellung von Kapseln aus dem fertigen Produkt wird ein Gleitmittel zugesetzt, z.B. Magnesiumstearat, ausgewählte Anteile 0,3 - 2 %, bevorzugte Anteile 0,4 - o,8 % des Fertigproduktes.

Die nach dem beschriebenen Verfahren erhältliche, in Form eines Copräzipitates vorliegenden Zubereitungen zeichnen sich durch eine gegenüber derjenigen der reinen Wirkstoffe erheblich erhöhte Freisetzungsrate und damit um eine wesentlich verbesserte Bioverfügbarkeit aus. Die Zubereitungen werden im Körper der mit ihnen behandelten Individuen ausgezeichnet resorbiert und entfalten die erwünschte und für das in Betracht kommende Flavano-Lignan erwartete physiologische Wirkung. Sie finden deshalb als Arzneimittel für die Behandlung von Lebererkrankungen und zur Prophylaxe von Leberschäden Verwendung.

### HERSTELLUNGSBEISPIELE

### HERSTELLUNG VON SILYMARIN-COPRÄZIPITAT

### A) Gewinnung von Silymarin (Silymarin I-IV)

Die Gewinnung von Silymarin wurde nach dem in der DE-C3-19 23 082, auf die hiermit Bezug genommen wird, beschriebenen Verfahren vorgenommen. Das dabei erhaltene Produkt wurde nach dem Verfahren gemäß der DE-C2-29 14 330, auf die hiermit Bezug genommen wird, aufgearbeitet und ergab Silymarin (Silymarin I-IV) mit einer mindestens 90%igen Reinheit.

### B) Herstellung der Basislösung

2,63 kg Mannitol
1,06 kg Natriumcarboxylmethylstärke
0,415 kg Polysorbat 80
12,895 kg Polyvidon
626 1 Ethanol (0,8)
Mannitol, Natriumcarboxylmethylstärke, Polysorbat 80 und Polyvidon wurden unter Rühren und Erwärmen in dem Ethanol gelöst. Die Einstellung des Ethanols auf die angegebene Dichte wurde mit reinem Wasser vorgenommen.

### C) Herstellung und Aufbereitung der Speiselösung

1. Bereitung der Lösung
   In die unter B hergestellte Basislösung wurden 18 kg nach dem unter A beschriebenen Verfahren hergestelltes Silymarin (berechnet auf Trockensubstanz) eindosiert. Das erhaltene Gemisch wurde bis zum Siedepunkt erwärmt, um eine klare Lösung zu erhalten. Die klare Lösung wurde über Filterschichten K 100 filtriert und in einer Vorlage zur Aufkonzentration bereitgestellt. Die Speiselösung wurde bei einer Temperatur > 45 °C gehalten.
2. Konzentrierung
   Die Speiselösung wurde unter Vakuum während
   50 Minuten in einer Verdampferanlage bei maximal 60 °C auf 1/10 des Volumens eingeengt.
3. Trocknung
   Das konzentrierte Material wurde in einem Vakuumtrockenschrank bei 70 °C und 1 mbar bis zu einer Restfeuchte < 7 % getrocknet.
4. Mahlung
   Das getrocknete Material wurde fein vermahlen (Soll-Siebrückstand > 40 µm muß < 1 % sein).
5. Nachtrocknung
   Die Nachtrocknung wurde in einer Vakuumtrocknungsanlage bei 1 mbar und 70 °C vorgenommen bis der limitierte Lösungsmittelanteil unterschritten worden war.

Das so hergestellte Copräzipitat wurde zur Bestimmung der Freisetzungsrate in vitro und zur Bestimmung der Bioverfügbarkeit in vivo untersucht, wie im folgenden noch beschrieben wird.

### HERSTELLUNG VON SILIBININ-COPRÄZIPITAT

### A) Gewinnung von isosilybinfreiem Silibinin

Die Gewinnung von isosilybinfreiem Silibinin wurde nach dem in der DE-C2-35 37 656, auf die hiermit Bezug genommen wird, beschriebenen Verfahren vorgenommen. Das dabei erhaltene Produkt enthält 97 bis 98,5 % Silibinin.

### B) Herstellung der Basislösungen I + II

### Basislösung I:

23,10 kg Polyvidon
und
2,316 kg Lactose
wurden in 375 l reinem Wasser suspendiert, worauf die Suspension unter Rühren und Erwärmen in eine klare Lösung überführt wurde.

### Basislösung II:

3,75 kg Silibinin (berechnet auf 100 % Silibinin) und
0,75 kg Polysorbat 80
wurden in 375 l Ethanol suspendiert, worauf die Suspension unter Erwärmen und Rühren in eine klare Lösung überführt wurde.

### C) Herstellung und Aufbereitung der Speiselösung

1. Bereitung der Lösung
   Die nach den unter B beschriebenen Basislösungen I und II wurden miteinander vereinigt, indem die Basislösung I unter Rühren in die Basislösung II eingemischt wurde. Dabei war darauf zu achten, daß eine klare Lösung entstand, die über Filterschichten K 100 filtriert und in einer Vorlage zur Aufkonzentration bereitgestellt wurde. Die Speiselösung wurde bei einer Temperatur > 45 °C gehalten.
2. Konzentrierung
   Die Speiselösung wurde unter Vakuum während 50 Minuten in einer Verdampferanlage bei maximal 60 °C auf 1/20 des Volumens eingeengt.
3. Trocknung
   Das konzentrierte Material wurde in einem Vakuumtrockenschrank bei 70 °C und 1 mbar bis zu einer Restfeuchte < 7 % getrocknet.
4. Mahlung
   Das getrocknete Material wurde fein vermahlen (Soll-Siebrückstand > 40 µm muß < 1 % sein).
5. Nachtrocknung
   Die Nachtrocklung wurde in einer Valcuumtrocknungsanlage bei 1 mbar und 70 °C vorgenommen bis der limitierte Lösungsmittelanteil unterschritten worden war.

Das so hergestellte Copräzipitat wurde zur Bestimmung der Freisetzungsrate in vitro und und zur Bestimmung der Bioverfügbarkeit in vivo untersucht, wie im folgenden noch beschrieben wird.

### BESTIMMUNG VON FREISETZUNGSRATE UND BIOVERFÜGBARKEIT VON SILYMARIN, SILYMARIN-COPRÄZIPITAT, SILIBININ UND SILIBININ-coPRÄZIPITAT

Die Bestimmung der Freisetzungsrate wurde in vitro mittels einer abgewandelten HPLC-Methode vorgenommen; die Bioverfügbarkeit wurde in vivo mittels klinischer Versuche bei gesunden Probanden ermittelt.

Die Versuchsergebnisse werden anhand der Figuren 1 bis 2 erläutert, dabei zeigen:
- Figur 1: einen Vergleich der Freisetzung von Silymarin mit der Freisetzung von Silymarin-Copräzipitat in Abhängigkeit von der Zeit (min) in schaubildlicher Darstellung;
- Figur 2: einen Vergleich der Freisetzung von Silibinin mit der Freisetzung von Silibinin-Copräzipitat in Abhängigkeit von der Zeit (min), in schaubildlicher Darstellung;

### Bestimmung der Freisetzungsrate von Silymarin, Silymarin-Copräzipitat, Silibinin und Silibinin-Copräzipitat in vitro

Zur Bestimmung der prozentualen Freisetzung von Silymarin-Copräzipitat im Vergleich zu reinem Silymarin wurden die beiden Substanzen in einer mit Blattrührer versehenen Apparatur in einer Pufferlösung (pH 7,5) bei 37 °C während 60 Minuten gerührt und anschließend zentrifugiert. Die Bestimmung der prozentualen Freisetzung wurde chromatographisch mittels einer neuentwickelten HPLC-Methode durchgeführt.

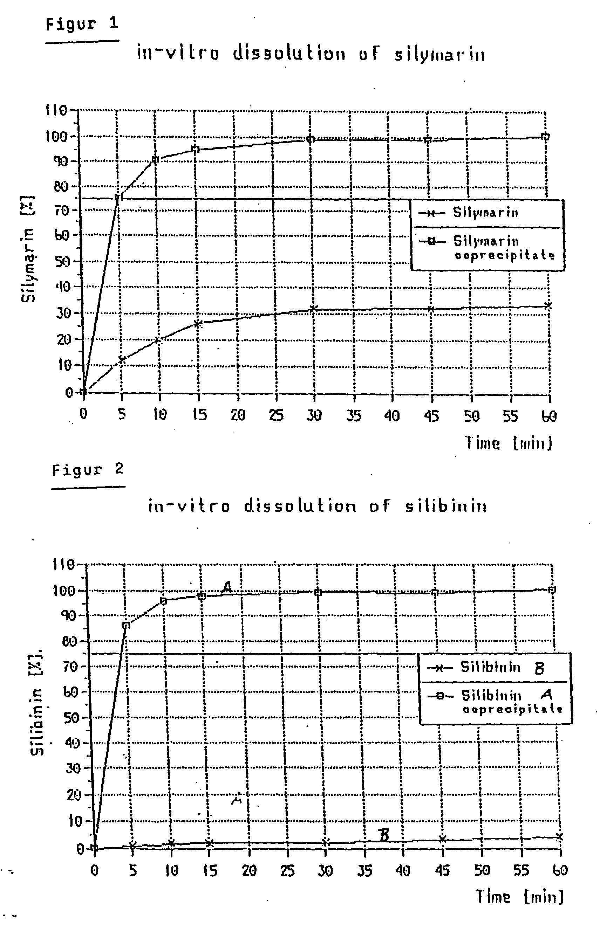

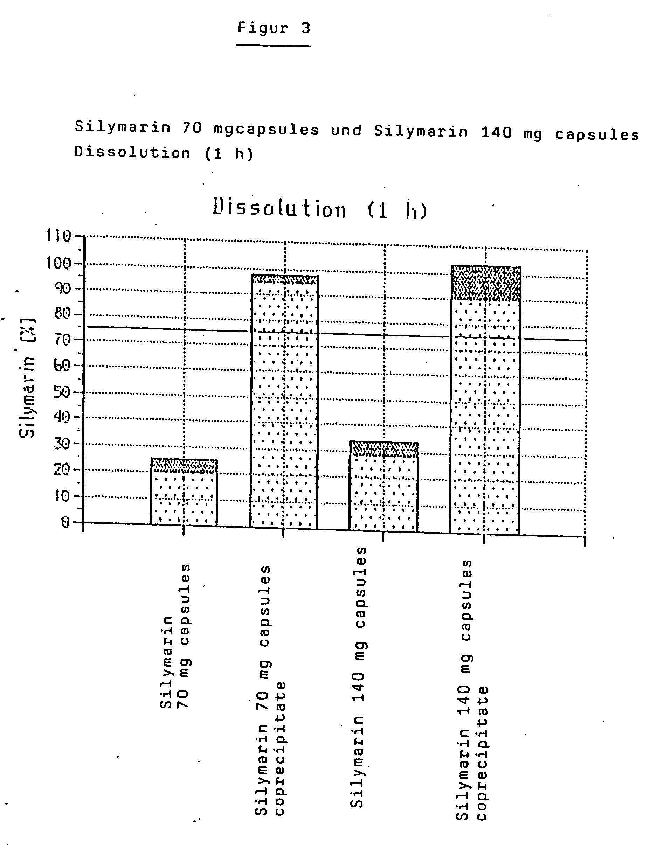

Aus Figur 3 ist deutlich ersichtlich, daß die prozentuale Freisetzung von Silymarin-Copräzipitat erheblich höher ist als diejenige von Silymarin in Form des reinen Wirkstoffes. Sie beträgt bei Silymarin in Form des reinen Wirkstoffes in 140 mg Kapseln annähernd 30 % bei einem Streubereich, durch die dunkle Schraffierung wiedergegeben, von < 10 %, wohingegen die prozentuale Freisetzung bei Silymarin-Copräzipitat annähernd 95 % -bei einem Streubereich von etwa 10 % beträgt. Diese Ergebnisse werden auch in den Freisetzungsprofilen von Silymarin und Silymarin-Copräzipitat mit der Zeit auf Figur 1 bestätigt.

Figur 2 zeigt die Freisetzungsprofile von Silibinin und Silibinin-Copräzipitat in Abhängigkeit mit der Zeit. Dabei ist auf der Abszisse die Zeit in Minuten und auf der Ordinate die Freisetzung in Prozenten angegeben. Die Versuchsdurchführung entsprach der im Zusammenhang mit der Bestimmung der prozentualen Freisetzung von silymarin und Silymarin-Copräzipitat beschriebenen. Aus dieser Figur ist ersichtlich, daß die prozentuale Freisetzung von Silibinin-Copräzipitat diejenige des reinen Wirkstoffes Silibinin um ein mehrfaches übersteigt. Die aus drei Meßreihen gewonnene Kurve (A), welche die Freisetzung des Silibinin-Copräzipitates wiedergibt, liegt - nach einem steilen Anstieg während der ersten fünf Minuten - oberhalb der 90 %-Markierung. Hingegen liegt die, ebenfalls aus drei Meßreihen gewonnene Kurve (B) für die Freisetzung des reinen Wirkstoffes Silibinin unterhalb der 10 %-Markierung, wobei die Freisetzungsrate von Silibinin nach 30 Minuten unterhalb 5 % liegt.

### Bestimmung der Bioverfügbarkeit von Silibinin und Silibinin-Copräzipitat in vivo

Die klinischen Versuche wurden im Gegensatz zu früheren Untersuchungen, die mit an Lebererkrankungen leidenden Probanden, beispielsweise cholezystektomierten Patienten mit Gallen-T-Drain oder bei Patienten mit außerhepatitischen Erkrankungen durchgeführt worden waren, mit willkürlich ausgewählten gesunden Freiwilligen als Probanden durchgeführt.

Bei den nachfolgend beschriebenen Untersuchungen wurden sechs gesunde männliche Probanden ausgewählt. Diesen wurden in nüchternem Zustand Einzeldosen von 102, 153, 203 und 254 mg Silibin verabreicht. Die Probanden wurden hospitalisiert, und zwar vom Abend vor der Medikation bis 50 Stunden nach der Verabreichung. Die Probanden erhielten während der Untersuchung Standardmahlzeiten und -getränke. Außerdem wurden sie dazu angehalten, während der Untersuchung einschließlich 24 Stunden vor der Medikation übermäßige körperliche Anstrengungen und andere Aktivitäten im Freien zu vermeiden sowie das Rauchen und die die Konsumierung xanthinhaltiqer Getränke, wie Kaffee, Tee und Schokolade zu unterlassen.

Die Charakteristika der Probanden und das Verabreichungsschema sind in der nachfolgenden Tabelle 1 wiedergegeben.

**TABELLE 1**

| Charakteristika der Probanden und Verabreichungsschema | | | | | | | |
|---|---|---|---|---|---|---|---|
| Proband | Alter (J) | Größe (cm) | Gewicht (kg) | Verabreichungsfolge Zahl der Kapseln | | | |
| 1 | 29 | 172 | 68 | 2 | 3 | 4 | 5 |
| 2 | 22 | 178 | 69 | 3 | 4 | 5 | 2 |
| 3 | 26 | 185 | 76 | 4 | 5 | 2 | 3 |
| 4 | 24 | 182 | 66 | 5 | 2 | 3 | 4 |
| 5 | 29 | 175 | 75 | 2 | 5 | 4 | 3 |
| 6 | 29 | 190 | 71 | 4 | 2 | 5 | 3 |

Die Bestimmung der Wirkstoffkonzentration im Plasma und der ausgeschiedenen Menge im Urin der Probanden wurde mittels einer spezifischen HPLC-Methode durchgeführt. Diese Methode zeichnete sich, wie bereits erwähnt, gegenüber den bisher bekannten durch eine wesentlich niedrigere Nachweisgrenze aus. Diese lag bei den bekannten Methoden bei annähernd 50 ng/ml im Plasma, während die Nachweisgrenze bei der neuen Methode bei einem um den Faktor 10 niedrigeren Wert liegt.

### Zusammenfassung der Versuchsergebnisse

Die Durchführung der klinischen Versuche und die Konzentrationsbestimmungen erfolgte wie im Vorhergehenden beschrieben und im Zusammenhang mit Tabelle 2 diskutiert. Die Ergebnisse sind in Tabelle 2 aufgeführt, wobei in dieser Tabelle die relative Bioverfügbarkeit von Silibinin derjenigen von Silibinin-Copräzipitat gegenübergestellt ist. Die relative Bioverfügbarkeit ist in [ng*h/ml] wiedergegeben.

**TABELLE 2**

| AUC [ng*h/ml] für Silibinin-Copräzipitat und Silibinin nach 4 oralen Gaben an gesunde Probanden | | |
|---|---|---|
| Proband Nr. | Silibinin-Copräzipitat | Silibinin |
| 1 | 1364 | 421,6 |
| 2 | 1589 | 617,6 |
| 3 | 2637 | 697,4 |
| 4 | 3004 | 710,6 |
| 5 | 2448 | 629,3 |
| 6 | 5355 | 1292,0 |
| 7 | 2254 | 642,5 |
| 8 | 1531 | 615,5 |
| MW | 2523 | 703,3 |
| AUC Relative Bioverfübarkeit (üblicherweise in der Pharmazeutik verwendete Abkürzung für: area under curve, entsprechend dem Integral des von einer Kurve begrenzten Bereiches). MW = Mittelwert | | |

Schädliche Wirkungen wurden nicht beobachtet, woraus hervorgeht, daß Silibinin-Copräzipitat ebenso wie Silibinin und Silymarin auch in hohen Dosierungen vertragen wird.

## Patentansprüche

1. Verfahren zur Herstellung einer Flavano-Lignan-Zubereitung **dadurch gekennzeichnet, daß** man a) eine wäßrig-alkoholische Lösung von pharmazeutisch annehmbaren Trägerstoffen und Netzmitteln herstellt, in dieser Lösung ein Flavano-Lignan suspendiert und das erhaltene Gemisch bis zur Siedetemperatur erwärmt, um eine klare Lösung zu bilden, oder b) ein Flavano-Lignan und ein Netzmittel in Alkohol suspendiert, die erhaltene Suspension unter Rühren bis zur Bildung einer klaren Lösung erwärmt, diese mit einer wäßrigen Lösung von pharmazeutisch annehmbaren Trägerstoffen versetzt und die erhaltene Mischung solange unter Rühren erwärmt, bis eine klare Lösung vorliegt, und daß man anschließend die nach a) oder b) erhaltene klare Lösung zur Bildung eines Copräzipitates konzentriert, filtriert und das entstandene Copräzipitat unter Vakuum trocknet.

2. Verfahren nach Anspruch 1, **dadurch gekennzeichnet, daß** man ein pulverförmiges Silymarin-Copräzipitat herstellt.

3. Verfahren nach Anspruch 1, **dadurch gekennzeichnet, daß** man ein pulverförmiges Silibinin-Copräzipitat herstellt.

4. Verfahren nach einem der Ansprüche 1 bis 3 **dadurch gekennzeichnet, daß** man als pharmazeutisch annehmbaren Trägerstoff in Wasser lösliche Zuckerderivate als Mono- und Disaccharide und/oder Cellulosederivate und als pharmazeutisch annehmbare Trägerstoffe für disperse Systeme lineare Polymere des 1-vinyl-2-pyrrolidon verwendet.

5. Verfahren nach einem der Ansprüche 1 bis 4, **dadurch gekennzeichnet, daß** man als Netzmittel Polysorbate oder Sorbitanester von Fettsäuren verwendet.

6. Verfahren nach Anspruch 4, **dadurch gekennzeichnet, daß** man als Zucker Mannitol oder Lactose verwendet.

7. Verfahren nach Anspruch 4, **dadurch gekennzeichnet daß** man die Cellulosederivate Natriumcarboxymethylstärke, Hydroxyethylstärke oder Carboxymethylcellulose verwendet.

8. Verfahren nach Anspruch 4, **dadurch gekennzeichnet, daß** man das lineare Polymer des 1-Vinyl-2-pyrrolidon Polyvidon verwendet.

9. Verfahren nach Anspruch 5, **dadurch gekennzeichnet, daß** man das Polysorbat 80 verwendet.

10. Flavano-Lignan-Zubereitung, als Copräzipitat mit pharmazeutisch annehmbaren Trägerstoffen und Netzmitteln, wie sie nach dem Verfahren nach einem der Ansprüche 1 bis 9 erhältlich ist.

11. Flavano-Lignan-Zubereitung nach Anspruch 10, **dadurch gekennzeichnet, daß** sie ein Silymarin-Copräzipitat enthält.

12. Flavano-Lignan-Zubereitung nach Anspruch 10, **dadurch gekennzeichnet, daß** sie ein Silibinin-Copräzipitat enthält.

13. Arzneimittel, enthaltend eine in Form eines Copräzipitates vorliegende Flavano-Lignan-Zubereitung, wie sie nach dem Verfahren nach Anspruch 1 erhältlich ist.

14. Arzneimittel, mitteln, enthaltend ein pulverförmiges Silymarin-Copräzipitat, wie es nach dem Verfahren nach Anspruch 2 erhältlich ist.

15. Arzneimittel, enthaltend ein pulverförmiges Silibinin-Copräzipitat, wie es nach dem Verfahren nach Anspruch 3 erhältlich ist.

16. Verfahren zur Herstellung eines Arzneimittels, zur prophylaktischen und/oder therapeutischen Behandlung von Lebererkrankungen, **dadurch gekennzeichnet, daß** man eine in Form eines Copräzipitates aus einem Flavano-Lignan und mindestens einem pharmazeutisch annehmbaren Trägerstoff vorliegende Flavano-Lignan-Zubereitung, wie sie nach dem Verfahren nach einem der Ansprüche 1 bis 5 erhältlich ist, zu einer für die orale Verabreichung geeigneten Dosierungsform konfektioniert.

## Claims

1. Process for producing a flavano-lignan preparation, **characterized in that** a) a hydroalcoholic solution of pharmaceutically acceptable carriers and wetting agents is prepared, a flavano-lignan is suspended in the solution, and the resulting mixture is heated to the boiling point in order to form a clear solution, or b) a flavano-lignan and a wetting agent are suspended in alcohol, the resulting suspension is heated with stirring until a clear solution forms, the latter is mixed with an aqueous solution of pharmaceutically acceptable carriers, and the resulting mixture is heated with stirring until a clear solution forms, and **in that** subsequently the clear solution obtained according to a) or b) is concentrated to form a coprecipitate, filtered and the resulting coprecipitate is dried under vacuum.

2. Process according to Claim 1, **characterized in that** a silymarin coprecipitate in powder form is produced.

3. Process according to Claim 1, **characterized in that** a silibinin coprecipitate in powder form is produced.

4. Process according to any of Claims 1 to 3, **characterized in that** water-soluble sugar derivatives as mono- and disaccharides and/or cellulose derivatives are used as pharmaceutically acceptable carrier, and linear polymers of 1-vinyl-2-pyrrolidone are used as pharmaceutically acceptable carriers for disperse systems.

5. Process according to any of Claims 1 to 4, **characterized in that** polysorbates or sorbitan esters of fatty acids are used as wetting agent.

6. Process according to Claim 4, **characterized in that** mannitol or lactose is used as sugar.

7. Process according to Claim 4, **characterized in that** the cellulose derivatives sodium carboxymethylstarch, hydroxyethylstarch or carboxymethylcellulose are used.

8. Process according to Claim 4, **characterized in that** the linear polymer of 1-vinyl-2-pyrrolidone polyvidone is used.

9. Process according to Claim 5, **characterized in that** polysorbate 80 is used.

10. Flavano-lignan preparation as coprecipitate with pharmaceutically acceptable carriers and wetting agents as is obtainable by the process according to any of Claims 1 to 9.

11. Flavano-lignan preparation according to Claim 10, **characterized in that** it comprises a silymarin coprecipitate.

12. Flavano-lignan preparation according to Claim 10, **characterized in that** it comprises a silibinin coprecipitate.

13. Medicament comprising a flavano-lignin preparation which is in the form of a coprecipitate and is obtainable by the process according to Claim 1.

14. Medicament comprising a silymarin coprecipitate in powder form as is obtainable by the process according to Claim 2.

15. Medicament comprising a silibinin coprecipitate in powder form as is obtainable by the process according to Claim 3.

16. Process for producing a medicament for the prophylactic and/or therapeutic treatment of hepatic disorders, **characterized in that** a flavano-lignan preparation which is in the form of a coprecipitate of a flavano-lignin and at least one pharmaceutically acceptable carrier and is obtainable by the process according to any of Claims 1 to 5 is processed to a osage form suitable for oral administration.

## Revendications

1. Procédé pour la production d'une préparation de flavonolignane, **caractérisé en ce que** a) on prépare une solution aqueuse-alcoolique de véhicules et d'agents mouillants pharmaceutiquement acceptables, on met un flavonolignane en suspension dans cette solution et on chauffe jusqu'à la température d'ébullition le mélange obtenu, pour obtenir une solution limpide, ou b) on met un flavonolignane et un agent mouillant en suspension dans de l'alcool, on chauffe sous agitation la suspension obtenue jusqu'à la formation d'une solution limpide, on y ajoute une solution aqueuse de véhicules pharmaceutiquement acceptables et on chauffe le mélange obtenu, sous agitation, jusqu'à l'obtention d'une solution limpide, et **en ce qu'**ensuite on concentre la solution limpide obtenue selon a) ou b), pour la formation d'un co-précipité, on filtre et on sèche sous vide le co-précipité résultant.

2. Procédé selon la revendication 1, **caractérisé en ce qu'**on prépare un co-précipité pulvérulent de silymarine.

3. Procédé selon la revendication 1, **caractérisé en ce qu'**on prépare un co-précipité pulvérulent de silibinine.

4. Procédé selon l'une quelconque des revendications 1 à 3, **caractérisé en ce qu'**on utilise en tant que véhicule pharmaceutiquement acceptable des dérivés de sucres solubles dans l'eau, sous forme de mono- et/ou disaccharides et/ou de dérivés de cellulose et, en tant que véhicules pharmaceutiquement acceptables pour des systèmes dispersés, des polymères linéaires de la 1-vinyl-2-pyrrolidone.

5. Procédé selon l'une quelconque des revendications 1 à 4, **caractérisé en ce qu'**on utilise en tant qu'agent mouillant des polysorbates ou des esters de sorbitanne avec des acides gras.

6. Procédé selon la revendication 4, **caractérisé en ce qu'**on utilise comme sucre le mannitol ou le lactose.

7. Procédé selon la revendication 4, **caractérisé en ce qu'**on utilise les dérivés de cellulose carboxyméthylamidon sodique, hydroxyéthylamidon ou carboxyméthylcellulose.

8. Procédé selon la revendication 4, **caractérisé en ce qu'**on utilise le polymère linéaire de la 1-vinyl-2-pyrrolidone polyvidone.

9. Procédé selon la revendication 5, **caractérisé en ce qu'**on utilise du Polysorbat 80.

10. Préparation de flavonolignane sous forme de co-précipité avec des véhicules pharmaceutiquement acceptables et des agents mouillants, tels qu'elle peut être obtenue conformément au procédé selon l'une quelconque des revendications 1 à 9.

11. Préparation de flavonolignane selon la revendication 10, **caractérisée en ce qu'**elle contient un co-précipité de silymarine.

12. Préparation de flavonolignane selon la revendication 10, **caractérisée en ce qu'**elle contient un co-précipité de silibinine.

13. Médicament contenant une préparation de flavonolignane se trouvant sous forme d'un co-précipité, telle qu'elle peut être obtenue conformément au procédé selon la revendication 1.

14. Médicament contenant un co-précipité pulvérulent de silymarine, tel qu'il peut être obtenu conformément au procédé selon la revendication 2.

15. Médicament contenant un co-précipité pulvérulent de silibinine, tel qu'il peut être obtenu conformément au procédé selon la revendication 3.

16. Procédé pour la fabrication d'un médicament destiné au traitement prophylactique et/ou thérapeutique d'hépatopathies, **caractérisé en ce qu'**on transforme en une forme galénique appropriée à l'administration orale une préparation de flavonolignane se trouvant sous forme d'un co-précipité à base d'un flavonolignane et d'au moins un véhicule pharmaceutiquement acceptable, telle qu'elle peut être obtenue conformément au procédé selon l'une quelconque des revendications 1 à 5.
